(19) Europäisches Patentamt
European Patent Office
Office européen des brevets

(11) Veröffentlichungsnummer: **0 447 645 A1**

(12) # EUROPÄISCHE PATENTANMELDUNG

(21) Anmeldenummer: **90124294.1**

(22) Anmeldetag: **15.12.90**

(51) Int. Cl.5: **C07D 251/22**, C07D 253/04, C07D 253/07, C07D 213/76, C07D 237/20, C07D 239/42, C07D 281/10, A01N 47/36

(30) Priorität: **17.03.90 DE 4008627**

(43) Veröffentlichungstag der Anmeldung: **25.09.91 Patentblatt 91/39**

(84) Benannte Vertragsstaaten: **BE CH DE ES FR GB IT LI NL**

(71) Anmelder: **BAYER AG**

**W-5090 Leverkusen 1 Bayerwerk(DE)**

(72) Erfinder: **Haas, Wilhelm, Dr.**
**Nordstrasse 1**
**W-5014 Kerpen 3(DE)**
Erfinder: **Kirsten, Rolf, Dr.**
**Carl-Langhans-Strasse 27**
**W-4019 Monheim(DE)**
Erfinder: **Kysela, Ernst, Dr.**
**Virchowstrasse 14**
**W-5060 Bergisch Gladbach 1(DE)**
Erfinder: **Santel, Hans-Joachim, Dr.**
**Grünstrasse 9a**
**W-5090 Leverkusen 1(DE)**
Erfinder: **Lürssen, Klaus, Dr.**
**August-Kierspel-Strasse 145**
**W-5060 Bergisch Gladbach 2(DE)**
Erfinder: **Schmidt, Robert Rudolf, Dr.**
**Im Waldwinkel 110**
**W-5060 Bergisch Gladbach 2(DE)**

(54) **Aralkylsulfonylharnstoffe.**

(57) Die Erfindung betrifft neue Aralkylsulfonylharnstoffe der Formel (I)

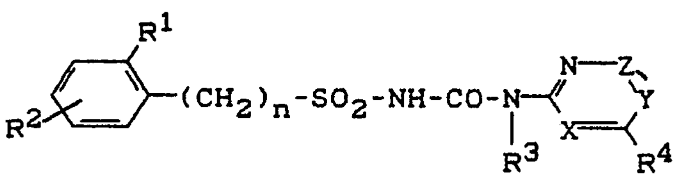

(I)

in welcher
n für die Zahlen 2 oder 3 steht,
X für N oder CH steht,
Y für N oder $CR^7$ steht,
Z für N oder $CR^8$ steht,
(wobei $R^1$, $R^2$, $R^3$, $R^4$, $R^7$ und $R^8$ die in der Beschreibung angegebenen Bedeutungen haben),
Verfahren und neue Zwischenprodukte zu ihrer Herstellung sowie ihre Verwendung als Herbizide.

EP 0 447 645 A1

Die Erfindung betrifft neue Aralkylsulfonylharnstoffe, Verfahren und neue Zwischenprodukte zu ihrer Herstellung sowie ihre Verwendung als Herbizide.

Es wurden neue Aralkylsulfonylharnstoffe der allgemeinen Formel (I)

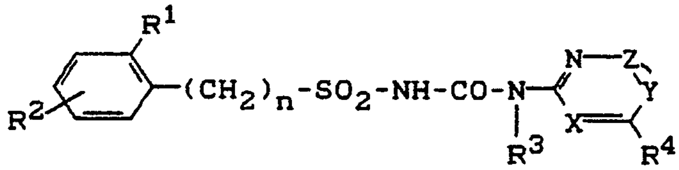

(I)

in welcher

n für die Zahlen 2 oder 3 steht,

$R^1$ und $R^2$ gleich oder verschieden sind und für Wasserstoff, Halogen, Carboxy, Cyano, Nitro, für jeweils gegebenenfalls durch Halogen substituiertes $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkoxy-carbonyl oder für die Gruppierung

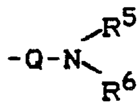

stehen,

worin

Q für Carbonyl (CO) oder Sulfonyl ($SO_2$) steht,

$R^5$ für Wasserstoff oder $C_1$-$C_4$-Alkyl steht und

$R^6$ für Wasserstoff, $C_1$-$C_4$-Alkyl oder $C_1$-$C_4$-Alkoxy steht,

$R^3$ für Wasserstoff, $C_1$-$C_4$-Alkyl, $C_3$-$C_4$-Alkenyl, $C_3$-$C_4$-Alkinyl, Benzyl, Cyano oder für ein Metalläquivalent steht,

$R^4$ für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, Bis-($C_1$-$C_2$-Alkoxy)-$C_1$-$C_2$-alkyl, $C_1$- $C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino, Dimethylamino oder Diethylamino steht,

X für Stickstoff oder eine -CH-Gruppierung steht,

Y für Stickstoff oder eine -$CR^7$-Gruppierung steht, worin

$R^7$ für Wasserstoff, Fluor, Chlor, Brom, Cyano, Methyl, Formyl, Acetyl, Methoxycarbonyl oder Ethoxycarbonyl steht, und

Z für Stickstoff oder eine -$CR^8$-Gruppierung steht, worin

$R^8$ für Wasserstoff, Fluor, Chlor, Brom, $C_1$-$C_4$-Alkyl, Trifluormethyl, $C_1$-$C_4$-Alkoxy, Difluormethoxy, $C_1$-$C_4$-Alkylthio, $C_1$-$C_4$-Alkylamino, Dimethylamino oder Diethylamino steht,

gefunden.

Man erhält die neuen Aralkylsulfonylharnstoffe der Formel (I), wenn man Arylalkansulfonamide der allgemeinen Formel (II)

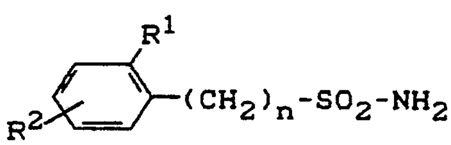

(II)

in welcher

n, $R^1$ und $R^2$ die oben angegebenen Bedeutungen haben,

oder reaktionsfähige Derivate von Verbindungen der Formel (II)

mit Aminoazinen der allgemeinen Formel (III)

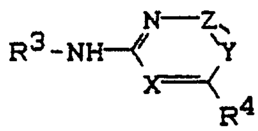

(III)

in welcher

$R^3$, $R^4$, X, Y und Z die oben angegebenen Bedeutungen haben,

oder mit reaktionsfähigen Derivaten von Verbindungen der Formel (III) gegebenenfalls in Gegenwart eines Reaktionshilfsmittels und gegebenenfalls in Gegenwart eines Verdünnungsmittels umsetzt, wobei wenigstens eine der Ausgangsverbindungen der Formeln (II) und (III) in Form eines reaktionsfähigen Derivates eingesetzt wird.

Die neuen Aralkylsulfonylharnstoffe der Formel (I) zeichnen sich durch starke herbizide Wirksamkeit aus.

Die neuen Aralkylsulfonylharnstoffe sind durch die Formel (I) allgemein definiert. Bevorzugt sind Verbindungen der Formel (I), worin

n für die Zahlen 2 oder 3 steht,

$R^1$ für Wasserstoff, Fluor, Chlor, Brom, Carboxy, Cyano, Nitro, für jeweils gegebenenfalls durch Fluor und/oder Chlor substituiertes $C_1$-$C_2$-Alkyl, $C_1$-$C_2$-Alkoxy, $C_1$-$C_2$-Alkylthio, $C_1$-$C_2$-Alkylsulfinyl, $C_1$-$C_2$-Alkylsulfonyl, $C_1$-$C_2$-Alkoxy-carbonyl oder für die Gruppierung

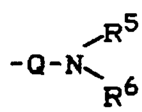

steht, worin

Q für Carbonyl (CO) oder Sulfonyl ($SO_2$) steht,

$R^5$ für Wasserstoff oder $C_1$-$C_2$-Alkyl steht und

$R^6$ für Wasserstoff, $C_1$-$C_2$-Alkyl oder $C_1$-$C_2$-Alkoxy steht,

$R^2$ für Wasserstoff, Fluor, Chlor, Brom, Carboxy, für gegebenenfalls durch Fluor und/oder Chlor substituiertes $C_1$-$C_2$-Alkyl oder $C_1$-$C_2$-Alkoxy oder für $C_1$-$C_2$-Alkoxy-carbonyl steht,

$R^3$ für Wasserstoff, Methyl, Natrium oder Kalium steht,

$R^4$ für Wasserstoff, Fluor, Chlor, Brom, Methyl, Methoxymethyl, Trifluormethyl, Methoxy, Ethoxy, Difluormethoxy, Methylthio, Ethylthio, Amino, Methylamino, Ethylamino, Dimethylamino oder Diethylamino steht,

X für Stickstoff oder eine -CH-Gruppierung steht,

Y für Stickstoff oder eine -$CR^7$-Gruppierung steht, worin

R7 für Wasserstoff, Fluor, Chlor oder Methyl steht, und

Z für Stickstoff oder eine -$CR^8$-Gruppierung steht, worin

$R^8$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino, Dimethylamino oder Diethylamino steht.

Besonders bevorzugt sind Aralkylsulfonylharnstoffe der Formel (I), in welcher

n für die Zahl 2 steht,

$R^1$ für Wasserstoff, Fluor, Chlor, Carboxy, Cyano, Nitro, Trifluormethyl, Difluormethoxy, Trifluormethoxy, Methoxycarbonyl oder Ethoxycarbonyl steht,

$R^2$ für Wasserstoff, Fluor, Chlor, Carboxy, Methyl, Trifluormethyl, Difluormethoxy, Trifluormethoxy oder Methoxycarbonyl steht,

$R^3$ für Wasserstoff steht,

$R^4$ für Wasserstoff, Methyl, Trifluormethyl, Methoxy, Ethoxy, Difluormethoxy, Methylthio oder Methylamino steht,

X für Stickstoff steht,

Y für Stickstoff oder eine CH-Gruppierung steht und

Z für eine -$CR^8$-Gruppierung steht, worin

$R^8$ für Wasserstoff, Chlor, Methyl, Ethyl, Trifluormethyl, Methoxy, Ethoxy oder Difluormethoxy steht.

Verwendet man für das erfindungsgemäße Verfahren beispielsweise 2-(2-Nitro-phenyl)-ethylsulfonylisocyanat und 2-Amino-4,6-dimethoxy-pyrimidin als Ausgangsstoffe, so kann der Reaktionsablauf durch das folgende Formelschema skizziert werden:

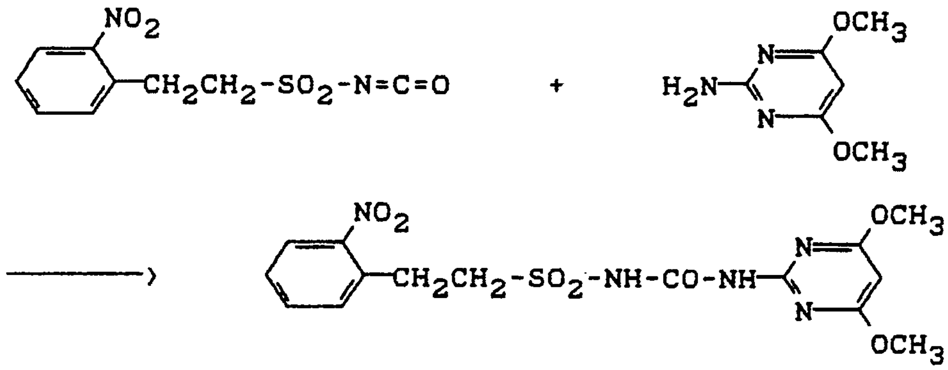

Die beim erfindungsgemäßen Verfahren zur Herstellung von Verbindungen der Formel (I) als Ausgangsstoffe zu verwendenden Arylalkansulfonamide sind durch die Formel (II) allgemein definiert.

In Formel (II) haben n, $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, $R^1$ und $R^2$ angegeben wurden.

Als Beispiele für die Ausgangsstoffe der Formel (II) seien genannt:

2-Phenyl-ethansulfonamid, 2-(2-Fluor-phenyl)-, 2-(2-Chlor-phenyl)-,2-(2-Brom-phenyl)-, 2-(3-Chlor-phenyl)-, 2-(3-Fluor-phenyl)-, 2-(2-Carboxy-phenyl)-, 2-(2-Cyano-phenyl)-,2-(3-Cyano-phenyl)-, 2-(2-Nitro-phenyl)-, 2-(2-Methyl-phenyl)-,2-(2-Trifluormethyl-phenyl)-, 2-(3-Trifluormethyl-phenyl)-, 2-(2-Methoxy-phenyl)-, 2-(2-Ethoxy-phenyl)-, 2-(2-Difluormethoxy-phenyl)-, 2-(2-Trifluormethoxy-phenyl)-, 2-(2-Methylthio-phenyl)-, 2-(2-Methylsulfinyl-phenyl)-, 2-(2-Methylsulfonyl-phenyl)-, 2-(2-Methoxycarbonyl-phenyl)-, 2-(3-Methoxycarbonyl-phenyl)-, 2-(2-Ethoxycarbonyl-phenyl)-, 2-(3-Ethoxycarbonyl-phenyl)-, 2-(2-Dimethylaminocarbonyl-phenyl)- und 2-(2-Dimethylaminosulfonyl-phenyl)-ethansulfonamid; 2-(4-Methyl-phenyl)-, 2-(2,6-Dimethyl-phenyl)-, 2-(2,5-Dichlor-phenyl)-, 2-(2,6-Dichlor-phenyl)-, 2-(4-Methoxycarbonyl-phenyl)-, 2-(4-Ethoxycarbonyl-phenyl)-, 2-(4-Carboxy-phenyl)-, 2-(4-Nitro-phenyl)-, 2-(4-Methoxy-phenyl)-, 2-(4-Trifluormethyl-phenyl)- und 2-(4-Cyano-phenyl)-ethansulfonamid; 3-(2-Chlor-phenyl)-, 3-(2-Carboxy-phenyl)-, 3-(2-Trifluormethyl-phenyl)-, 3-(2-Difluormethoxy-phenyl)-, 3-(2-Trifluormethoxy-phenyl)- und 3-(2-Methoxycarbonyl-phenyl)-propansulfonamid.

Die Ausgangsstoffe der Formel (II) sind bekannt und/oder können nach an sich bekannten Verfahren hergestellt werden (vgl. J. Med. Chem. 6 (1963), 506-508; J. Org. Chem. 30 (1965), 3163-3166; J. Am. Chem. Soc. 103 (1981), 1525-1533; J. Org. Chem. 49 (1984), 5124-5131; J. Org. Chem. 50 (1985), 2066-2073).

Neu und ebenfalls Gegenstand der vorliegenden Erfindung sind die Arylalkansulfonamide der Formel (IIa)

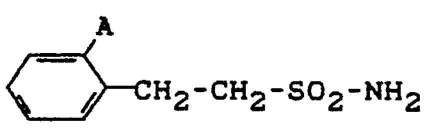

(IIa)

in welcher

A für Trifluormethyl, Difluormethoxy, Trifluormethoxy, Methoxycarbonyl, Ethoxycarbonyl, Carboxy oder Cyano steht.

Man erhält die neue Verbindung der Formel (IIa), in welcher A für Trifluormethyl steht, wenn man das bekannte 2-(2-Trifluormethyl-phenyl)-ethylchlorid [2-(2-Chlorethyl)-α,α,α-trifluortoluol oder 2-(2-Chlorethyl)-benzotrifluorid; Firma Aldrich] mit Benzylmercaptan in Gegenwart eines Säureakzeptors, wie z.B. Kaliumhydroxid, und in Gegenwart eines Verdünnungsmittels, wie z.B. Methanol, Ethanol, Propanol, Isopropanol und Wasser, bei Temperaturen zwischen 0° C und 120° C umsetzt, anschließend einengt und das im Rückstand erhaltene 2-(2-Benzylthioethyl)-benzotrifluorid der Formel (IVa)

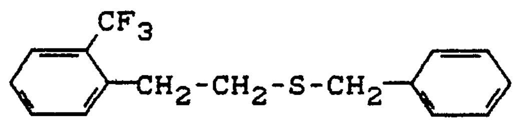

(IVa)

mit Chlor in Gegenwart eines Verdünnungsmittels, wie z.B. Essigsäure, und in Gegenwart von Wasser bei Temperaturen zwischen 0° C und 20° C umsetzt, nach üblichen Methoden aufarbeitet (vgl. die Herstellungsbeispiele) und das hierbei erhaltene 2-(2-Trifluormethyl-phenyl)-ethansulfonylchlorid der Formel (IVb)

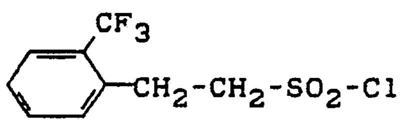

(IVb)

mit Ammoniak in Gegenwart eines Verdünnungsmittels, wie z.B. Tetrahydrofuran, Dioxan und/oder Wasser, bei Temperaturen zwischen 0° C und 50° C umsetzt und nach üblichen Methoden aufarbeitet (vgl. die Herstellungsbeispiele).

Man erhält die neuen Verbindungen der Formel (IIa), in welcher A für Difluormethoxy oder Trifluormethoxy steht, wenn man 2-Difluormethoxy-benzylchlorid oder 2-Trifluormethoxy-benzylchlorid (vgl. DE-OS 2150399, DE-OS 2150955, DE-OS 3642824) mit Natriumcyanid oder Kaliumcyanid in Gegenwart eines Verdünnungsmittels, wie z.B. Methanol, Ethanol und/oder Wasser, bei Temperaturen zwischen 0° C und 100° C umsetzt und nach üblichen Methoden aufarbeitet (vgl. die Herstellungsbeispiele), die hierbei erhaltenen Produkte - 2-Difluormethoxy-benzylcyanid oder 2-Trifluormethoxy-benzylcyanid - im Autoklaven mit Wasserstoff und Ammoniak in Gegenwart eines Katalysators, wie z.B. Raney-Cobalt, und in Gegenwart eines Verdünnungsmittels, wie z.B. Methanol oder Ethanol, bei Temperaturen zwischen 0° C und 150° C und bei Drücken zwischen 50 bar und 150 bar umsetzt und nach üblichen Methoden aufarbeitet (vgl. die Herstellungsbeispiele), die hierbei erhaltenen Produkte - 2-(2-Difluormethoxy-phenyl)-ethylamin oder 2-(2-Trifluormethoxy-phenyl)-ethylamin - mit Natriumnitrit oder Kaliumnitrit in Gegenwart von Wasser und in Gegenwart einer Säure, wie z.B. Essigsäure, bei Temperaturen zwischen -20° C und +100° C umsetzt und nach üblichen Methoden aufarbeitet (vgl. die Herstellungsbeispiele), die hierbei erhaltenen Produkte - 2-(2-Difluormethoxy-phenyl)-ethanol oder 2-(2-Trifluormethoxy-phenyl)-ethanol - mit einem Sulfonsäurechlorid, wie z.B. Methansulfonylchlorid, in Gegenwart eines Säureakzeptors, wie z.B. Triethylamin, und in Gegenwart eines Verdünnungsmittels, wie z.B. Dichlormethan, bei Temperaturen zwischen 0° C und 50° C umsetzt und nach üblichen Methoden aufarbeitet, die so erhaltenen Sulfonylierungsprodukte mit Benzylmercaptan in Gegenwart eines Säureakzeptors, wie z.B. Kaliumhydroxid oder Natriumhydroxid, und in Gegenwart eines Verdünnungsmittels, wie z.B. Ethanol und/oder Isopropanol, bei Temperaturen zwischen 0° C und 100° C umsetzt und nach üblichen Methoden aufarbeitet (vgl. die Herstellungsbeispiele), und die hierbei erhaltenen Produkte - Difluormethoxy-2-(2-benzylthioethyl)-benzol und Trifluormethoxy-2-(2-benzylthioethyl)-benzol - nach der oben beschriebenen Methodik in entsprechende Sulfonsäurechloride - 2-(2-Difluormethoxy-phenyl)-ethansulfonylchlorid und 2-(2-Trifluormethoxy-phenyl)-ethansulfonylchlorid - und schließlich in entsprechende Sulfonsäureamide - 2-(2-Difluormethoxy-phenyl)-ethansulfonamid und 2-(2-Trifluormethoxy-phenyl)-ethansulfonamid - überführt (vgl. die Herstellungsbeispiele).

Das gemäß obiger Beschreibung erhaltene 2-(2-Trifluormethyl-phenyl)-ethansulfonamid (IIa, A = $CF_3$) wird zur Herstellung der weiteren neuen Verbindungen der Formel (IIa) zunächst mit Aluminiumchlorid in Gegenwart eines Verdünnungsmittels, wie z.B. Dichlormethan, bei Temperaturen zwischen 0° C und 50° C umgesetzt; dann wird hydrolysiert und nach üblichen Methoden aufgearbeitet (vgl. die Herstellungsbeispiele).

Die hierbei erhaltene Verbindung der Formel (V)

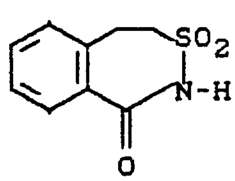

(V)

kann dann wie folgt weiter umgewandelt werden:

(a) Umsetzung mit Wasser, Methanol oder Ethanol, gegebenenfalls in Gegenwart eines Reaktionshilfsmittels, wie z.B. Salzsäure, bei Temperaturen zwischen 20° C und 100° C, führt zu den neuen Verbindungen der Formel (IIa), bei denen A für Carboxy, Methoxycarbonyl oder Ethoxycarbonyl steht;

(b) Umsetzung von (V) mit Phosphorylchlorid ($POCl_3$) bei Temperaturen zwischen 0° C und 100° C führt zum 2-(2-Cyano-phenyl)-ethansulfonylchlorid, welches mit Ammoniak auf übliche Weise zur Verbindung der Formel (IIa), in welcher A für Cyano steht, umgewandelt werden kann.

In bevorzugten Varianten des erfindungsgemäßen Herstellungsverfahrens für die Aralkylsulfonylharnstoffe der Formel (I) werden entweder reaktionsfähige Derivate von Verbindungen der Formel (II) mit Verbindungen der Formel (III) oder Verbindungen der Formel (II) mit reaktionsfähigen Derivaten von Verbindungen der Formel (III) umgesetzt. Bevorzugte Derivate sind jeweils entsprechende Isocyanate oder entsprechende Urethane (Carbamate).

Bevorzugte reaktionsfähige Derivate von Verbindungen der Formel (II) sind die Isocyanate der Formel (IIb) und die Urethane (Carbamate) der Formel (IIc)

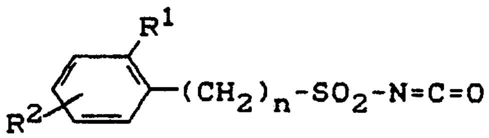

(IIb)

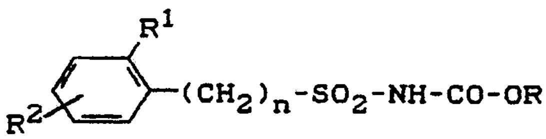

(IIc)

wobei

n, $R^1$ und $R^2$ vorzugsweise bzw. insbesondere diejenigen Bedeutungen haben, die oben für die Verbindungen der Formel (I) vorzugsweise bzw. als insbesondere bevorzugt für n, $R^1$ und $R^2$ angegeben wurden und

R vorzugsweise für Methyl, Ethyl, Benzyl oder Phenyl, insbesondere für Phenyl steht.

Die Verbindungen der Formeln (IIb) und (IIc) sind noch nicht aus der Literatur bekannt und sind gleichfalls Gegenstand der vorliegenden Erfindung.

Man erhält die neuen Isocyanate der Formel (IIb) aus entsprechenden Arylalkansulfonamiden der Formel (II) - oben - nach üblichen Methoden, beispielsweise wenn man zunächst durch Umsetzung mit einem Alkylisocyanat, wie z.B. Butylisocyanat, gegebenenfalls in Gegenwart eines Katalysators, wie z.B. Diazabicyclo-[2.2.2]-octan (DABCO), bei Temperaturen zwischen 100° C und 150° C entsprechende Sulfonylharnstoffe erzeugt und diese im angegebenen Temperaturbereich mit Phosgen umsetzt (vgl. z.B. EP-A 51466/US-P 4420325), oder auch wenn man Verbindungen der Formel (II) mit Chlorsulfonylisocyanat, gegebenenfalls in Gegenwart eines Katalysators, wie z.B. Diazabicyclo-[2.2.2]-octan (DABCO), und gegebenenfalls in Gegenwart eines Verdünnungsmittels, wie z.B. Chlorbenzol, bei Temperaturen zwischen 50° C und 130° C umsetzt (vgl. z.B. DE-OS 3228101).

Die neuen Urethane der Formel (IIc) erhält man aus entsprechenden Arylalkansulfonamiden der Formel (II) ebenfalls nach üblichen Methoden, beispielsweise durch Umsetzung mit Chlorameisensäureestern, wie z.B. Chlorameisensäure-methylester, -ethylester, -benzylester oder -phenylester, in Gegenwart eines Säureakzeptors, wie z.B. Natriumhydrid oder Kalium-tert-butylat, und gegebenenfalls in Gegenwart eines Verdünnungsmittels, wie z.B. Tetrahydrofuran, bei Temperaturen zwischen 0° C und 100° C (vgl. EP-A 101407).

Die beim erfindungsgemäßen Verfahren zur Herstellung von Verbindungen der Formel (I) weiter als Ausgangsstoffe zu verwendenden Aminoazine sind durch die Formel (III) allgemein definiert.

In Formel (III) haben $R^3$, $R^4$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^3$, $R^4$, X, Y und Z angegeben wurden.

Als Beispiele für die Ausgangsstoffe der Formel (III) seien genannt:

4,6-Dimethyl-, 4-Methoxy-6-methyl-, 4,6-Dimethoxy-, 4-Ethoxy-6-methyl-, 4-Ethyl-6-methoxy-, 4,6-Diethoxy-, 4-Chlor-6-methoxy-, 4-Methyl-, 4-Chlor-6-methyl-, 4-Methyl-6-methylthio-, 4-Methyl-6-methylamino-, 4-Methoxy-6-trifluormethyl-, 4-Chlor-6-trifluormethyl-, 4-Difluormethoxy-6-methyl-, 4-Chlor-6-difluormethoxy- und 4,6-Bis-difluormethoxy-2-amino-pyrimidin; 4,6-Dimethyl-, 4-Methoxy-6-methyl-, 4,6-Dimethoxy-, 4-Ethoxy-6-methyl-, 4-Ethyl-6-methoxy- und 4,6-Diethoxy-2-amino-s-triazin; 4,6-Dimethyl-, 4-Methoxy-6-methyl-, 4,6-Dimethoxy-, 4-Ethoxy-6-methyl-, 4-Ethyl-6-methoxy- und 4,6-Diethoxy-2-methylamino-s-triazin.

Die Verbindungen der Formel (III) sind bekannt und/oder können nach an sich bekannten Verfahren

hergestellt werden (vgl. Chem. Pharm. Bull. 11 (1963), 1382; US-P 4299960; EP-A 121082; EP-A 125205; EP-A 126711; EP-A 152378).

Bevorzugte reaktionsfähige Derivate von Verbindungen der Formel (III) sind die Isocyanate der Formel (IIIa) und die Urethane (Carbamate) der Formel (IIIb)

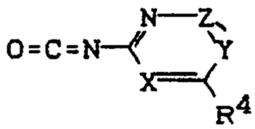

(IIIa)

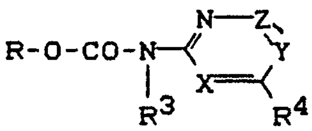

(IIIb)

wobei

| | |
|---|---|
| $R^3$, $R^4$, X, Y und Z | vorzugsweise bzw. insbesondere diejenigen Bedeutungen haben, die oben für die Verbindungen der Formel (I) vorzugsweise bzw. als insbesondere bevorzugt für $R^3$, $R^4$, X, Y und Z angegeben wurden und |
| R | vorzugsweise für Methyl, Ethyl, Benzyl oder Phenyl, insbesondere für Phenyl steht. |

Die Verbindungen der Formeln (IIIa) und (IIIb) sind bekannt und/oder können nach an sich bekannten Verfahren hergestellt werden (vgl. Angew. Chem. 83 (1971), 406-408; DE-OS 1943635; CH-P 579062; US-P 3919228; EP-A 30140; EP-A 101670).

Das erfindungsgemäße Verfahren zur Herstellung der neuen Aralkylsulfonylharnstoffe der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren wird gegebenenfalls in Gegenwart eines Reaktionshilfsmittels durchgeführt. Hierzu gehören insbesondere aliphatische, aromatische oder heterocyclische Amine, wie Triethylamin, N,N-Dimethylanilin, Pyridin, 2-Methyl-5-ethyl-pyridin, 4-Dimethylamino-pyridin, Diazabicyclooctan (DABCO), Diazabicyclononen (DBN) und Diazabicycloundecen (DBU).

Die Reaktionstemperaturen können bei dem erfindungsgemäßen Verfahren in einem größeren Bereich variiert werden. Im allgemeinen arbeitet man bei Temperaturen zwischen 0 °C und 120 °C, vorzugsweise bei Temperaturen zwischen 10 °C und 80 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel, gegebenenfalls in Gegenwart eines Reaktionshilfsmittels, durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die Verbindungen der Formel (I) eignen sich insbesondere zur selektiven Bekämpfung von dikotylen Unkräutern in monokotylen Kulturen, vor allem im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5-(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); N-Phosphonomethyl-glycin (GLYPHOSATE); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 4-Ethylamino-2-t-butylamino-6-methylthio-s-triazin(TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

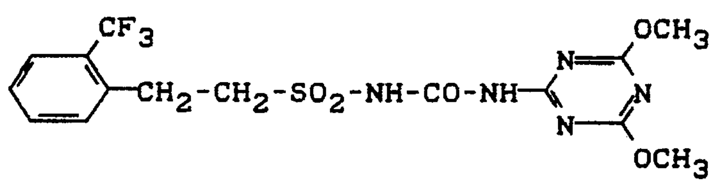

Eine Lösung von 5,06 g (20 mMol) 2-(2-Trifluormethylphenyl)-ethansulfonamid in 50 ml Chlorbenzol wird auf 90° C erwärmt und bei dieser Temperatur werden 3,1 g (22 mMol) Chlorsulfonylisocyanat innerhalb von 30 Minuten eindosiert. Das Reaktionsgemisch wird weitere 2 Stunden bei 90° C bis 100° C gerührt und anschließend im Ölpumpenvakuum eingeengt. Das im Rückstand verbliebene 2-(2-Trifluormethyl-phenyl)-ethylsulfonylisocyanat wird in 50 ml Acetonitril aufgenommen und mit 3,12 g (20 mMol) 2-Amino-4,6-dimethoxy-s-triazin versetzt. Das Reaktionsgemisch wird 12 Stunden bei 20° C gerührt, dann in 250 ml Wasser eingerührt, mit konzentrierter Salzsäure auf pH 1 eingestellt und mit Essigsäureethylester extrahiert. Die organische Phase wird mit Natriumsulfat getrocknet und filtriert; vom Filtrat wird das

Lösungsmittel im Wasserstrahlvakuum sorgfältig abdestilliert.

Man erhält 1,68 g (19% der Theorie) 1-(4,6-Dimethoxy-s-triazin-2-yl)-3-[2-(2-trifluormethyl-phenyl)-ethylsulfonyl]-harnstoff vom Schmelzpunkt 145° C.

Beispiel 2

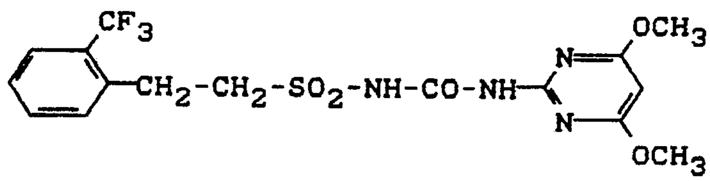

5,06 g (20 mMol) 2-(2-Trifluormethyl-phenyl)-ethansulfonamid werden - wie in Beispiel 1 beschrieben - mit 3,1 g (22 mMol) Chlorsulfonylisocyanat umgesetzt. Nach Entfernen des Chlorbenzols wird der Rückstand in 50 ml Acetonitril aufgenommen und mit 3,1 g (20 mMol) 2-Amino-4,6-dimethoxy-pyrimidin versetzt. Nach Zugabe von 0,2 g 1,4-Diazabicyclo-[2.2.2]-octan (DABCO) wird die Reaktionsmischung 14 Stunden bei 50° C gerührt und dann in 250 ml Wasser eingerührt. Das nach Ansäuern mit Salzsäure kristallin anfallende Produkt wird durch Absaugen isoliert.

Man erhält 3,1 g (36% der Theorie) 1-(4,6-Dimethoxy-pyrimidin-2-yl)-3-[2-(2-trifluormethyl-phenyl)-ethylsulfonyl]-harnstoff vom Schmelzpunkt 167° C.

Analog zu den Beispielen 1 und 2 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

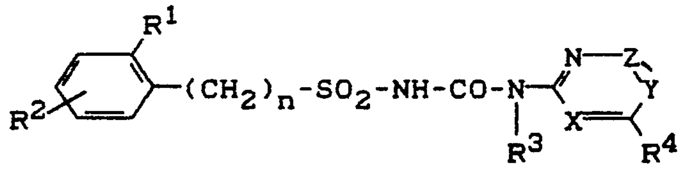

(I)

Tabelle 1: Beispiele für die Verbindungen der Formel (I)

| Bsp.-Nr. | n | $R^1$ | $R^2$ | $R^3$ | $R^4$ | X | Y | Z | Schmelzpunkt (°C) |
|---|---|---|---|---|---|---|---|---|---|
| 3 | 2 | $CF_3$ | H | H | $CH_3$ | N | CH | $C-CH_3$ | 125 |
| 4 | 2 | $CF_3$ | H | H | $OCH_3$ | N | N | $C-CH_3$ | 118 |
| 5 | 2 | $CF_3$ | H | H | $CH_3$ | N | CH | CH | 206 |
| 6 | 2 | $CF_3$ | H | H | $CH_3$ | N | N | $C-CH_3$ | 139 |
| 7 | 2 | $COOCH_3$ | H | H | $OCH_3$ | N | CH | $C-OCH_3$ | 151 |
| 8 | 2 | $COOCH_3$ | H | H | $OCH_3$ | N | N | $C-OCH_3$ | 126 |
| 9 | 2 | CN | H | H | $OCH_3$ | N | CH | $C-OCH_3$ | 175 |
| 10 | 2 | $NO_2$ | H | H | $OCH_3$ | N | CH | $C-OCH_3$ | 173 |
| 11 | 2 | $NO_2$ | H | H | $CH_3$ | N | CH | $C-CH_3$ | 82 |
| 12 | 2 | COOH | H | H | $OCH_3$ | N | CH | $C-OCH_3$ | 205 |
| 13 | 2 | $COOC_2H_5$ | H | H | $CH_3$ | N | CH | $C-CH_3$ | 138 |
| 14 | 2 | CN | H | H | $CH_3$ | N | CH | $C-CH_3$ | 137 |
| 15 | 2 | $COOCH_3$ | H | H | $CH_3$ | N | CH | $C-CH_3$ | 152 |
| 16 | 2 | $COOC_2H_5$ | H | H | $OCH_3$ | N | CH | $C-OCH_3$ | 148 |
| 17 | 3 | $OCF_3$ | H | H | $CH_3$ | N | CH | $C-CH_3$ | Öl |

Tabelle 1: - Fortsetzung -

| Bsp.-Nr. | n | $R^1$ | $R^2$ | $R^3$ | $R^4$ | X | Y | Z | Schmelzpunkt (°C) |
|---|---|---|---|---|---|---|---|---|---|
| 18 | 2 | $CF_3$ | H | H | $OCH_3$ | N | CH | $C-CH_3$ | 130 |
| 19 | 2 | CN | H | H | $OCH_3$ | N | CH | $C-CH_3$ | 183 |
| 20 | 2 | $NO_2$ | H | H | $OCH_3$ | N | CH | $C-CH_3$ | 158 |
| 21 | 2 | $NO_2$ | H | H | $OCH_3$ | N | N | $C-OCH_3$ | 158 |
| 22 | 2 | $NO_2$ | H | H | $CH_3$ | N | CH | CH | 195 |
| 23 | 2 | $NO_2$ | H | H | $CH_3$ | N | N | $C-OCH_3$ | 135 |
| 24 | 2 | $NO_2$ | H | H | $CH_3$ | N | N | $C-CH_3$ | 185 |
| 25 | 2 | $NO_2$ | H | $CH_3$ | $OCH_3$ | N | N | $C-OCH_3$ | 117 |
| 26 | 2 | $COOC_2H_5$ | H | H | $OCH_3$ | N | CH | $C-CH_3$ | 155 |
| 27 | 2 | $COOCH_3$ | H | H | $OCH_3$ | N | CH | $C-CH_3$ | 160 |
| 28 | 2 | $COOC_2H_5$ | H | H | $CH_3$ | N | CH | CH | 189 |
| 29 | 2 | CN | H | H | $CH_3$ | N | CH | CH | 208 |
| 30 | 2 | $COOCH_3$ | H | H | $CH_3$ | N | CH | CH | 175 |
| 31 | 2 | CN | H | H | $OCH_3$ | N | N | $C-OCH_3$ | 170 |
| 32 | 2 | CN | H | H | $OCH_3$ | N | N | $C-CH_3$ | 175 |
| 33 | 2 | CN | H | H | $CH_3$ | N | N | $C-CH_3$ | 160 |
| 34 | 2 | CN | H | $CH_3$ | $OCH_3$ | N | N | $C-OCH_3$ | 164 |
| 35 | 2 | $COOCH_3$ | H | $CH_3$ | $OCH_3$ | N | N | $C-OCH_3$ | 101 |
| 36 | 2 | $COOC_2H_5$ | H | H | $OCH_3$ | N | N | $C-OCH_3$ | 145 |
| 37 | 2 | $CF_3$ | H | $CH_3$ | $OCH_3$ | N | N | $C-OCH_3$ | 108 |

Tabelle 1: - Fortsetzung -

| Bsp.-Nr. | n | $R^1$ | $R^2$ | $R^3$ | $R^4$ | X | Y | Z | Schmelzpunkt (°C) |
|---|---|---|---|---|---|---|---|---|---|
| 38 | 2 | H | (3-)$CF_3$ | H | $OCH_3$ | N | CH | C-$OCH_3$ | 110 |
| 39 | 2 | H | (4-)F | H | $OCH_3$ | N | CH | C-$OCH_3$ | 148 |
| 40 | 2 | F | H | H | $OCH_3$ | N | CH | C-$OCH_3$ | 159 |
| 41 | 2 | H | (4-)Cl | H | $OCH_3$ | N | CH | C-$OCH_3$ | 172 |
| 42 | 2 | H | (4-)Cl | H | $OCH_3$ | N | CH | C-$CH_3$ | 147 |
| 43 | 2 | F | H | H | $OCH_3$ | N | CH | C-$CH_3$ | 119 |
| 44 | 2 | H | (3-)$CF_3$ | H | $OCH_3$ | N | CH | C-$CH_3$ | 88 |
| 45 | 2 | H | (4-)F | H | $OCH_3$ | N | CH | C-$CH_3$ | 120 |
| 46 | 2 | H | (3-)$CF_3$ | H | $OCH_3$ | N | N | C-$OCH_3$ | 133 |
| 47 | 2 | H | (4-)F | H | $OCH_3$ | N | N | C-$OCH_3$ | 148 |
| 48 | 2 | H | (4-)F | H | $OCH_3$ | N | N | C-$CH_3$ | 143 |
| 49 | 2 | H | (4-)Cl | H | $OCH_3$ | N | N | C-$OCH_3$ | 136 |
| 50 | 2 | $CF_3$ | H | H | $SCH_3$ | N | N | C-$OCH_3$ | 162 |
| 51 | 2 | $CF_3$ | H | H | $SCH_3$ | N | N | C-$CH_3$ | 172 |
| 52 | 2 | $CF_3$ | H | H | $SCH_3$ | N | N | C-$N(CH_3)_2$ | 167 |
| 53 | 2 | $CF_3$ | H | Na | $OCH_3$ | N | N | C-$OCH_3$ | 140 |
| 54 | 2 | $CF_3$ | H | Na | $OCH_3$ | N | CH | C-$OCH_3$ | (amorph) |
| 55 | 2 | $OCF_3$ | H | H | $CH_3$ | N | CH | C-$CH_3$ | 134 |
| 56 | 2 | $OCHF_2$ | (5-)Cl | H | $OCH_3$ | N | CH | C-$OCH_3$ | 143 |
| 57 | 2 | $OCHF_2$ | (5-)Cl | H | $CH_3$ | N | CH | C-$CH_3$ | 156 |

Tabelle 1: - Fortsetzung -

| Bsp.-Nr. | n | $R^1$ | $R^2$ | $R^3$ | $R^4$ | X | Y | Z | Schmelzpunkt (°C) |
|---|---|---|---|---|---|---|---|---|---|
| 58 | 2 | $SO_2N(CH_3)_2$ | H | H | $OCH_3$ | N | CH | $C-OCH_3$ | 175 |
| 59 | 2 | $OCF_3$ | H | H | $OCH_3$ | N | CH | $C-CH_3$ | 124 |
| 60 | 2 | $OCHF_2$ | H | H | $OCH_3$ | N | N | $C-OCH_3$ | 126 |
| 61 | 2 | $OCF_3$ | H | H | $OCH_3$ | N | CH | $C-OCH_3$ | 157 |
| 62 | 2 | $OCHF_2$ | H | H | $OCH_3$ | N | CH | $C-OCH_3$ | 171 |

Die Herstellung der als Beispiel 7 aufgeführten Verbindung nach einer weiteren Variante des erfindungsgemäßen Verfahrens ist nachstehend beispielhaft beschrieben:

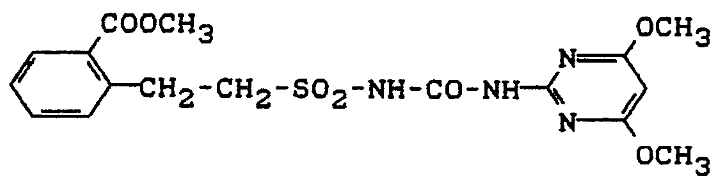

Eine Mischung aus 4,86 g (20 mMol) 2-(2-Methoxy-carbonyl-phenyl)-ethansulfonamid, 5,5 g (20 mMol) 4,6-Dimethoxy-2-phenoxycarbonylamino-pyrimidin, 3,04 g (20 mMol) 1,8-Diazabicyclo[5.4.0]undec-7-en (DBU) und 50 ml Acetonitril wird 12 Stunden bei 20°C gerührt. Dann wird filtriert, das Filtrat in 250 ml Wasser eingerührt und mit Salzsäure auf pH 1 eingestellt. Das kristallin anfallende Produkt wird durch Absaugen isoliert.

Man erhält 4,66 g (55% der Theorie) 1-(4,6-Dimethoxypyrimidin-2-yl)-3-[2-(2-methoxycarbonyl-phenyl)-ethylsulfonyl]-harnstoff vom Schmelzpunkt 151° C.

Ausgangsstoffe der Formel (IIa)

Beispiel (IIa-1)

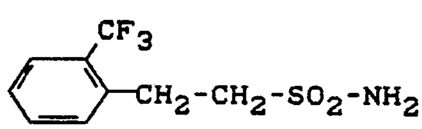

Stufe 1:

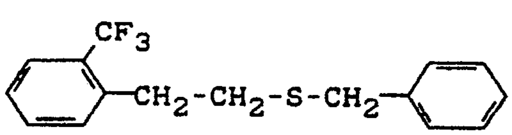

313 g (1,5 Mol) 2-(2-Chlorethyl)-benzotrifluorid (Firma Aldrich) werden in 2000 ml Isopropanol gelöst und nacheinander mit 187 g (1,5 mol) Benzylmercaptan und 95 g (1,7 Mol) Kaliumhydroxid - gelöst in 300

ml Ethanol/Wasser (70:30) - versetzt. Man erhitzt 16 Stunden zum Rückfluß und destilliert das Isopropanol im Vakuum ab. Das im Rückstand enthaltene 2-(2-Benzylthioethyl)-benzotrifluorid wird ohne weitere Reinigung in die nächste Reaktion eingesetzt.

$^1$H-NMR (300 MHz, $CDCl_3$):

$\delta$ = 2,65 (m, 2H); 3,05 (m, 2H); 3,75 (s, 2H); 7,2-7,7 (m,9H) ppm.

Stufe 2:

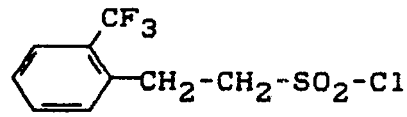

444 g (1,5 Mol) 2-(2-Benzylthioethyl)-benzotrifluorid werden in 2000 ml Eisessig gelöst und bis zur beginnenden Trübung mit Wasser versetzt (ca. 250 ml). Bei einer Temperatur von 10-12° C leitet man Chlorgas bis zur Sättigung ein und rührt 2 Stunden bei Raumtemperatur (20° C) nach. Überschüssiges Chlorgas wird danach unter leichtem Vakuum entfernt und das Reaktionsgemisch mit 8 l Wasser verdünnt. Der nach Extraktion mit Essigester, Trocknen der organischen Phase und Entfernen des Lösungsmittels anfallende ölige Rückstand wird im Vakuum über eine 50 cm Vigreux-Kolonne destilliert.

Man erhält 298 g (73% der Theorie) 2-(2-Trifluormethylphenyl)-ethansulfonylchlorid als farblosen Feststoff vom Schmelzpunkt 25-26° C und Siedepunkt 115-117° C/1,5 mbar.

$^1$H-NMR (300 MHz, $CDCl_3$):

$\delta$ = 3,52 (m, 2H); 3,90 (m, 2H); 7,3-7,8 (m, 4H) ppm.

Stufe 3:

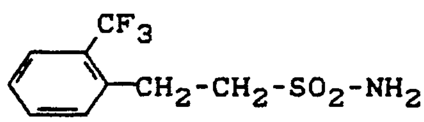

408,0 g (1,5 Mol) 2-(2-Trifluormethylphenyl)-ethansulfonylchlorid werden in 2000 ml abs. Tetrahydrofuran gelöst. Bei Raumtemperatur werden 52,7 g (3,1 Mol) Ammoniak eingeleitet. Man rührt 18 Stunden bei Raumtemperatur, gießt das Reaktionsgemisch auf 15 l Wasser und saugt den Feststoff ab. Nach Waschen mit Petrolether und Trocknen erhält man 354,2 g (93% der Theorie) 2-(2-Trifluormethylphenyl)-ethansulfonamid als weißen Feststoff vom Schmelzpunkt 120-121° C.

$^1$H-NMR (300 MHz, DMSO-$D_6$):

$\delta$ = 3,23 (m, 4H); 7,03 (s, 2H); 7,4-7,8 (m, 4H) ppm.

Beispiel (IIa-2)

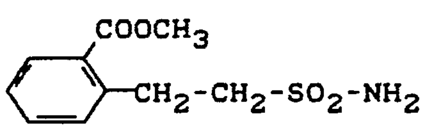

Stufe 1:

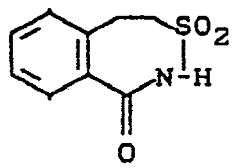

126,5 g (0,5 Mol) 2-(2-Trifluormethylphenyl)-ethansulfonamid werden in 500 ml abs. Dichlormethan gelöst und portionsweise unter Eiskühlung mit 133,5 g (1 Mol) Aluminiumchlorid versetzt. Man rührt 14 h bei Raumtemperatur (20° C), hydrolysiert vorsichtig mit 1000 ml Eiswasser und rührt weitere 60 Stunden bei Raumtemperatur. Man trennt die Phasen und extrahiert die wäßrige Phase mehrmals mit Dichlormethan. Die organischen Phasen werden vereinigt, mit 5%iger Natriumhydrogencarbonat-Lösung gewaschen und getrocknet. Nach Entfernen des Lösungsmittels erhält man 99 g (94% der Theorie) des Produktes obiger Struktur als weißen Feststoff vom Schmelzpunkt 186-187° C.

$^1$H-NMR ($CDCl_3$, 300 MHz):

$\delta$ = 3,36 (t, 2H); 3,77 (t, 2H); 7,2-7,8 (m, 4H); 8,51 (s, br, 1H) ppm.

Stufe 2:

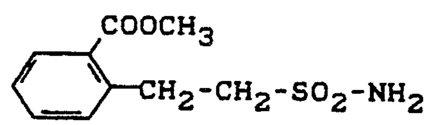

52,8 g (0,25 Mol) des Produktes aus Stufe 1 werden in 1500 ml Methanol gelöst. Nach Zugabe von 500 ml konzentrierter Salzsäure erhitzt man 2 Stunden unter Rückfluß. Man entfernt anschließend ca. zwei Drittel des eingesetzten Methanols, verdünnt den Rückstand mit 2000 ml Wasser und isoliert den anfallenden weißen Feststoff durch Absaugen. Nach Trocknen erhält man 43,3 g (71% der Theorie) 2-(2-Methoxycarbonylphenyl)-ethansulfonamid vom Schmelzpunkt 149-151° C.

$^1$H-NMR (300 MHz, $CDCl_3$):

$\delta$ = 3,48 (s, 4H); 3,90 (s, 3H); 4,94 (s, br, 2H); 7,3-8,0 (m, 4H) ppm.

Beispiel (IIa-3)

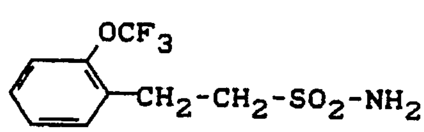

Stufe 1:

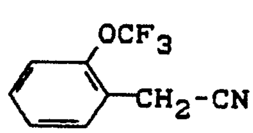

105 g (0,5 Mol) 2-Trifluormethoxy-benzylchlorid werden bei 25° C innerhalb von 2 Stunden in eine Mischung von 100 ml Ethanol, 25 ml Wasser und 30 g (0,6 Mol) Natriumcyanid (NaCN) getropft. Anschließend läßt man 5 Stunden unter Rückfluß sieden (80-90° C). Dann wird das Ethanol weitgehend abdestilliert und der Rückstand in 250 ml Dichlormethan aufgenommen; diese Dichlormethan-Lösung wird zunächst mit Wasser gewaschen, dann getrocknet und schließlich fraktioniert.

Man erhält 86 g (81 % der Theorie) 2-Trifluormethoxy-benzylcyanid (95 %ig); Siedepunkt 106-107°

C/18 mbar.

Stufe 2:

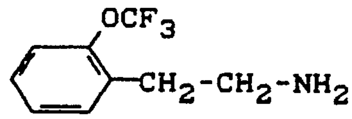

In einem Autoklaven wird eine Lösung von 100,5 g (0,5 Mol) 2-Trifluormethoxy-benzylcyanid in 350 ml Methanol vorgelegt. Nach Zugabe von 20 g Raney-Kobalt verschließt man die Apparatur und drückt 200 ml flüssiges Ammoniak ($NH_3$) und anschließend Wasserstoff bis zu einem Druck von 200 bar auf; man hält den Autoklaven 5 Stunden bei einer Temperatur von 80° C und einem $H_2$-Druck von 100-200 bar. Nach dem Abkühlen, Entspannen und Absaugen des Katalysators wird die Reaktionslösung fraktioniert.

Man erhält 73 g (70 % der Theorie) 2-(2-Trifluormethoxy-phenyl)-ethylamin (99 %ig), Siedepunkt 90-91° C/20 mbar.

Stufe 3:

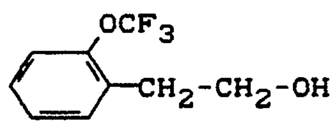

11,8 g (0,057 Mol) 2-(2-Trifluormethoxy-phenyl)-ethylamin werden in 35 ml 50 %iger Essigsäure gelöst; diese Lösung wird auf 0° C abgekühlt. Bei 0-5° C läßt man innerhalb von 15 Minuten eine Lösung von 4,1 g (0,06 Mol) Natrimnitrit ($NaNO_2$) in 10 ml Wasser zutropfen, erwärmt dann auf 80° C und läßt noch 1 Stunde bei dieser Temperatur nachrühren. Nach dem Abkühlen auf Raumtemperatur wird das Reaktionsgemisch eingeengt und der Rückstand mit 100 ml Dichlormethan und 100 ml Wasser versetzt und durchgerührt. Die organische Phase wird abgetrennt, über Natriumsulfat getrocknet, filtriert und eingeengt. Zu dem verbleibenden Rückstand gibt man 100 ml Ethanol und 60 ml 1N Kaliumhydroxidlösung und kocht 12 Stunden unter Rückfluß. Nach dem Abkühlen und Einengen wird der Rückstand mit Wasser und Dichlormethan versetzt und durchgerührt. Die organische Phase wird abgetrennt, mit Wasser gewaschen, über Natriumsulfat getrocknet und eingeengt.

Als Rückstand verbleiben 8,0 g (68 % der Theorie) 2-(2-Trifluormethoxy-phenyl)-ethanol als farbloses Öl.

$^1$H-NMR ($CDCl_3$, TMS):
$\delta$ = 2,95 (t, 2H); 3,85 (t, 2H); 7,2-7,35 (m, 4H)ppm.

Stufe 4:

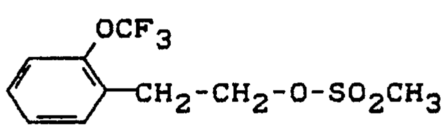

6,8 g (0,033 Mol) 2-(2-Trifluormethoxy-phenyl)-ethanol und 5,5 ml (0,04 Mol) Triethylamin werden in 100 ml Dichlormethan gelöst; diese Lösung wird auf 0° C abgekühlt. Bei 0-5° C werden 4,6 g (0,04 Mol) Methansulfonylchlorid zugetropft. Man läßt das Reaktionsgemisch auf Raumtemperatur kommen und rührt 6 Stunden bei ca. 20° C nach. Dann wird mit 1N-Salzsäure gewaschen, die Dichlormethanphase abgetrennt, über Natriumsulfat getrocknet, filtriert und eingeengt.

Als Rückstand verbleiben 9,3 g (99 % der Theorie) Methansulfonsäure-[2-(2-Trifluormethoxy-phenyl)-ethyl]-ester als gelbliches Öl.

$^1$H-NMR ($CDCl_3$, TMS):

δ = 2,90 (s, 3H); 3,14 (t, J = 6,8 Hz, 2H); 4,42 (t, J = 6,8 Hz, 2H); 7,25-7,35 (m, 4H)ppm.

Stufe 5:

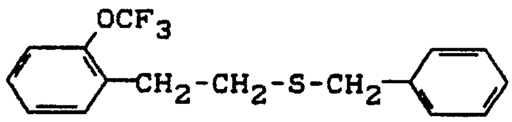

8,2 g (0,029 Mol) Methansulfonsäure-[2-(2-Trifluormethoxy-phenyl)-ethyl]-ester und 3,8 g (0,031 Mol) Benzylmercaptan werden in 50 ml Isopropanol gelöst und dazu wird bei Raumtemperatur eine Lösung von 1,9 g (0,033 Mol) Kaliumhydroxid in 8 ml 70 %igem, wäßrigem Ethanol getropft. Das Reaktionsgemisch wird 12 Stunden unter Rückfluß gekocht und anschließend eingeengt. Der Rückstand wird mit Essigsäureethylester aufgenommen und mit Wasser gewaschen. Man trennt die organische Phase ab, trocknet über Natriumsulfat, filtriert und engt ein.

Als Rückstand erhält man 9,0 g (98 % der Theorie) Benzyl-[2-(2-trifluormethoxy-phenyl)-ethyl]-thioether in Form eines rötlichen Öls.

$^1$H-NMR ($CDCl_3$, TMS):

δ = 2,63 (m, 2H); 2,90 (m, 2H); 3,75 (s, 2H); 7,15-7,33 (m, 9H)ppm.

Stufe 6:

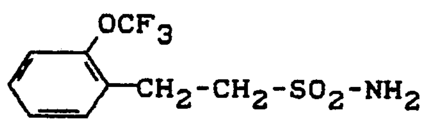

7,8 g (0,025 Mol) Benzyl-[2-(2-Trifluormethoxy-phenyl)-ethyl]-thioether werden in 40 ml Eisessig und 4 ml Wasser vorgelegt. Die Suspension wird auf 10° C abgekühlt und mit Chlorgas gesättigt. Man rührt noch 1 Stunde bei 20° C nach, verdünnt das Reaktionsgemisch mit 50 ml Wasser und extrahiert mit 100 ml Dichlormethan. Die Dichlormethanphase wird abgetrennt, über Natriumsulfat getrocknet, filtriert und eingeengt. Den verbleibenden Rückstand versetzt man mit 150 ml 25 %igem Ammoniak-Wasser und rührt 3 Stunden bei 20° C. Das kristalline Produkt wird abgesaugt, mit Wasser gewaschen und getrocknet und anschließend mit Petrolether verrührt, abgesaugt und wieder getrocknet. Man erhält 3,8 g (56 % der Theorie) 2-(2-Trifluormethoxy-phenyl)-ethansulfonamid als farblose Kristalle vom Schmelzpunkt 92° C.

Anwendungsbeispiele:

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen beispielsweise die Verbindungen gemäß den Herstellungsbeispielen 1, 2 und 4 bei guter Verträglichkeit gegenüber Nutzpflanzen, wie z.B. Weizen, starke Wirkung gegen Unkräuter, wie z.B. Amaranthus, Chenopodium, Datura, Polygonum und Sinapis.

## Patentansprüche

1. Aralkylsulfonylharnstoffe der allgemeinen Formel (I),

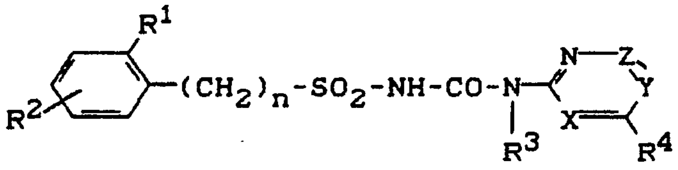

(I)

in welcher

n für die Zahlen 2 oder 3 steht,

$R^1$ und $R^2$ gleich oder verschieden sind und für Wasserstoff, Halogen, Carboxy, Cyano, Nitro, für jeweils gegebenenfalls durch Halogen substituiertes $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkoxy-carbonyl oder für die Gruppierung

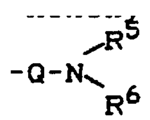

stehen,
worin

Q für Carbonyl (CO) oder Sulfonyl ($SO_2$) steht,

$R^5$ für Wasserstoff oder $C_1$-$C_4$-Alkyl steht und

$R^6$ für Wasserstoff, $C_1$-$C_4$-Alkyl oder $C_1$-$C_4$-Alkoxy steht,

$R^3$ für Wasserstoff, $C_1$-$C_4$-Alkyl, $C_3$-$C_4$-Alkenyl, $C_3$-$C_4$-Alkinyl, Benzyl, Cyano oder für ein Metalläquivalent steht,

$R^4$ für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, Bis-($C_1$-$C_2$-Alkoxy)-$C_1$-$C_2$-alkyl, $C_1$- $C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino, Dimethylamino oder Diethylamino steht,

X für Stickstoff oder eine -CH-Gruppierung steht,

Y für Stickstoff oder eine -$CR^7$-Gruppierung steht, worin

$R^7$ für Wasserstoff, Fluor, Chlor, Brom, Cyano, Methyl, Formyl, Acetyl, Methoxycarbonyl oder Ethoxycarbonyl steht, und

Z für Stickstoff oder eine -$CR^8$-Gruppierung steht, worin

$R^8$ für Wasserstoff, Fluor, Chlor, Brom, $C_1$ -$C_4$-Alkyl, Trifluormethyl, $C_1$-$C_4$-Alkoxy, Difluormethoxy, $C_1$-$C_4$-Alkylthio, $C_1$-$C_4$-Alkylamino, Dimethylamino oder Diethylamino steht.

2. Aralkylsulfonylharnstoffe der allgemeinen Formel (I) gemäß Anspruch 1, dadurch gekennzeichnet, daß darin

n für die Zahlen 2 oder 3 steht,

$R^1$ für Wasserstoff, Fluor, Chlor, Brom, Carboxy, Cyano, Nitro, für jeweils gegebenenfalls durch Fluor und/oder Chlor substituiertes $C_1$-$C_2$-Alkyl, $C_1$-$C_2$-Alkoxy, $C_1$-$C_2$-Alkylthio, $C_1$-$C_2$-Alkylsulfinyl, $C_1$-$C_2$-Alkylsulfonyl, $C_1$-$C_2$-Alkoxy-carbonyl oder
für die Gruppierung

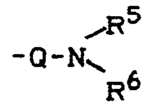

steht,
worin

- Q für Carbonyl (CO) oder Sulfonyl ($SO_2$) steht,
- $R^5$ für Wasserstoff oder $C_1$-$C_2$-Alkyl steht und
- $R^6$ für Wasserstoff, $C_1$-$C_2$-Alkyl oder $C_1$-$C_2$-Alkoxy steht,
- $R^2$ für Wasserstoff, Fluor, Chlor, Brom, Carboxy, für gegebenenfalls durch Fluor und/oder Chlor substituiertes $C_1$-$C_2$-Alkyl oder $C_1$-$C_2$-Alkoxy oder für $C_1$-$C_2$-Alkoxy-carbonyl steht,
- $R^3$ für Wasserstoff, Methyl, Natrium oder Kalium steht,
- $R^4$ für Wasserstoff, Fluor, Chlor, Brom, Methyl, Methoxymethyl, Trifluormethyl, Methoxy, Ethoxy, Difluormethoxy, Methylthio, Ethylthio, Amino, Methylamino, Ethylamino, Dimethylamino oder Diethylamino steht,
- X für Stickstoff oder eine -CH-Gruppierung steht,
- Y für Stickstoff oder eine -$CR^7$-Gruppierung steht, worin
- $R^7$ für Wasserstoff, Fluor, Chlor oder Methyl steht, und
- Z für Stickstoff oder eine -$CR^8$-Gruppierung steht, worin
- $R^8$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino, Dimethylamino oder Diethylamino steht.

**3.** Aralkylsulfonylharnstoffe der allgemeinen Formel (I) gemäß Anspruch 1, dadurch gekennzeichnet, daß darin

- n für die Zahl 2 steht,
- $R^1$ für Wasserstoff, Fluor, Chlor, Carboxy, Cyano, Nitro, Trifluormethyl, Difluormethoxy, Trifluormethoxy, Methoxycarbonyl oder Ethoxycarbonyl steht,
- $R^2$ für Wasserstoff, Fluor, Chlor, Carboxy, Methyl, Trifluormethyl, Difluormethoxy, Trifluormethoxy oder Methoxycarbonyl steht,
- $R^3$ für Wasserstoff steht,
- $R^4$ für Wasserstoff, Methyl, Trifluormethyl, Methoxy, Ethoxy, Difluormethoxy, Methylthio oder Methylamino steht,
- X für Stickstoff steht,
- Y für Stickstoff oder eine CH-Gruppierung steht und
- Z für eine -$CR^8$-Gruppierung steht, worin
- $R^8$ für Wasserstoff, Chlor, Methyl, Ethyl, Trifluormethyl, Methoxy, Ethoxy oder Difluormethoxy steht.

**4.** Verfahren zur Herstellung von Aralkylsulfonylharnstoffen der Formel (I) gemäß Anspruch 1, dadurch gekennzeichnet, daß man Arylalkansulfonamide der allgemeinen Formel (II)

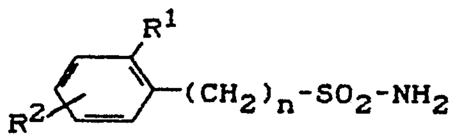

(II)

in welcher

n, $R^1$ und $R^2$ die in Anspruch 1 angegebenen Bedeutungen haben,

oder reaktionsfähige Derivate von Verbindungen der Formel (II)

mit Aminoazinen der allgemeinen Formel (III)

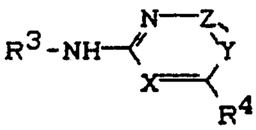

(III)

in welcher

$R^3$, $R^4$, X, Y und Z die in Aspruch 1 angegebenen Bedeutungen haben,

oder mit reaktionsfähigen Derivaten von Verbindungen der Formel (III)

gegebenenfalls in Gegenwart eines Reaktionshilfsmittels und gegebenenfalls in Gegenwart eines Verdünnungsmittels umsetzt, wobei wenigstens eine der Ausgangsverbindungen der Formeln (II) und (III) in Form eines reaktionsfähigen Derivates eingesetzt wird.

**5.** Herbizide Mittel, gekennzeichnet durch einen Gehalt an mindestens einem Aralkylsulfonylharnstoff der Formel (I) gemäß Anspruch 1.

**6.** Verfahren zur Bekämpfung von Unkräutern, dadurch gekennzeichnet, daß man Aralkylsulfonylharnstoffe der Formel (I) gemäß Anspruch 1 auf Unkräuter oder ihren Lebensraum einwirken läßt.

**7.** Verwendung von Aralkylsulfonylharnstoffen der Formel (I) gemäß Anspruch 1 zur Bekämpfung von Unkräutern.

**8.** Verfahren zur Herstellung von herbiziden Mitteln, dadurch gekennzeichnet, daß man Aralkylsulfonylharnstoffe der Formel (I) gemäß Anspruch 1 mit Streckmitteln und/oder oberflächenaktiven Mitteln vermischt.

**9.** Arylalkansulfonamide der Formel (IIa),

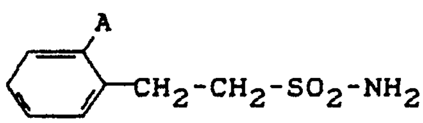

(IIa)

in welcher

A für Trifluormethyl, Difluormethoxy, Trifluormethoxy, Methoxycarbonyl, Ethoxycarbonyl, Carboxy oder Cyano steht.

**10.** Verbindung der Formel (V)

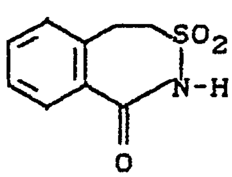

(V).

Europäisches Patentamt

# EUROPÄISCHER RECHERCHENBERICHT

Nummer der Anmeldung

EP 90124294.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | US - A - 4 599 412 (SANDELL) * Anspruch 1 * | 1,5-8 | C 07 D 251/22 C 07 D 253/04 C 07 D 253/07 C 07 D 213/76 C 07 D 237/20 C 07 D 239/42 C 07 D 281/10 C 07 C A 01 N 47/36 |
| A | US - A - 4 420 325 (SAUERS) * Ansprüche 1,20-38 * | 1,5-8 | |
| A | EP - A2 - 0 203 825 (DU PONT) * Ansprüche 1,14-16; Tabellen III-V * | 1,5-8 | |
| A | DE - B - 1 032 734 (HOECHST) * Beispiele 1,2 * | 9 | |
| D,A | EP - A2 - 0 030 140 (DU PONT) * Anspruch 1 * | 1,5-8 | |
| | | | RECHERCHIERTE SACHGEBIETE (Int Cl 5) |
| D,A | EP - B1 - 0 126 711 (CIBA-GEIGY) * Ansprüche 1,12-16 * | 1,5-8 | C 07 D 251/00 C 07 D 253/00 C 07 D 213/00 C 07 D 237/00 C 07 D 239/00 C 07 D 281/00 |
| A | CHEMICAL ABSTRACTS, Band 86, Nr. 3, 17. Januar 1977, Columbus, Ohio, USA ORAZI, ORFEO O. et al. "Intramolecular sulfonyl-amidomethylation; a new route to fused heterocyclic compounds" Seite 424, Spalte 1, Zusammenfassung-Nr. 16 646b & J. Chem. Soc. Chem. Commun. 1976, (12),470-1 | 10 | |
| A | CHEMICAL ABSTRACTS, Band 81, Nr. 15, 14. Oktober 1974, | 10 | |

Der vorliegende Recherchenbericht wurde für alle Patentansprüche erstellt.

| Recherchenort | Abschlußdatum der Recherche | Prüfer |
|---|---|---|
| WIEN | 02-05-1991 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

-2-

EP 90124294.1

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
| | Columbus, Ohio, USA<br>BOEHME, HORST et al.<br>"Isochroman and isothiochroman-1-yl azide"<br>Seite 462, Spalte 1, Zusammenfassung-Nr. 91 486z<br>& Justus Liebigs Ann. Chem. 1974, (5),734-40<br>-- | | |
| A | CHEMICAL ABSTRACTS, Band 103, Nr. 3, 22. Juli 1985,<br>Columbus, Ohio, USA<br>ABRAMOVITCH, RUDOLPH A. et al.<br>"Solution and flash vacuum pyrolysis of some 2,6-disubstituted beta-phenethylsulfonyl azides and of beta-styrenesulfonyl azide"<br>Seite 528, Spalte 1, Zusammenfassung-Nr. 22 049v<br>& J. Org. Chem. 1985, 50(12), 2066-73<br>-- | 9 | RECHERCHIERTE SACHGEBIETE (Int Cl 5) |
| X | CHEMICAL ABSTRACTS, Band 59, Nr. 11, 25. November 1963,<br>Columbus, Ohio, USA<br>BERNARD LOEV et al.<br>"Synthesis of some new sulfonylureas"<br>Spalte 12684, Zusammenfassung-Nr. 12 684h<br>& J. Med. Chem. 6(5), 506-8 (1963)<br>---- | 9 | |

Der vorliegende Recherchenbericht wurde für alle Patentansprüche erstellt.

| Recherchenort | Abschlußdatum der Recherche | Prüfer |
|---|---|---|
| WIEN | 02-05-1991 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument